# EUROPEAN PATENT SPECIFICATION

(11) **EP 1 018 884 B1**
(45) Date of publication and mention of the grant of the patent: **26.10.2005**
(21) Application number: 98906485.2
(22) Date of filing: 12.02.1998
(51) Int. Cl.: A01N 63/00, A61P 25/00, A61P 27/02

(54) **METHOD OF TREATING BLINDNESS WITH hNT HUMAN NEURONAL CELLS**
VERFAHREN ZUR BEHANDLUNG VON BLINDHEIT MIT MENSCHLICHEN NEURONALEN hNT ZELLEN
METHODE DE TRAITEMENT DE LA CECITE AVEC DES CELLULES NEURONALES HUMAINES hNT

(30) Priority: 12.02.1997 US 800224
(43) Date of publication of application: 19.07.2000
(73) Proprietor: Layton Bioscience, Inc., Sunnyvale, CA 94086 (US)
(72) Inventor: SNABLE, Gary, L., Atherton, CA 94027 (US)
(74) Representative: Roques, Sarah Elizabeth
(86) International application number: PCT/US1998/003019
(87) International publication number: WO 1998/034485

(56) References cited:
- DEL CERRO ET AL.: "INTRARETINAL GRAFTS OF DIFFERENTIATED HUMAN RETINOBLASTOMA CELLS INTEGRATE WITH THE HOST RETINA" BRAIN RESEARCH, vol. 583, 1992, pages 12-22, XP001068594 ELSEVIER SCIENCE LTD.
- DILORETO D ET AL: "CYCLOSPORINE TREATMENT PROMOTES SURVIVAL OF HUMAN FETAL NEURAL RETINA TRANSPLANTED TO THE SUBRETINAL SPACE OF THE LIGHT-DAMAGED FISCHER 344 RAT" EXPERIMENTAL NEUROLOGY, SAN DIEGO, CA, US, vol. 140, July 1996 (1996-07), pages 37-42, XP002911862
- CERRO DEL M ET AL: "NEITHER INTRAOCULAR GRAFTS OF RETINAL CELL HOMOGENATES NOR LIVE NON-RETINAL NEURONS PRODUCE BEHAVIORAL RECOVERY IN RATS WITH LIGHT-DAMAGED RETINAS" CELL TRANSPLANTATION, ELSEVIER SCIENCE, US, vol. 4, no. 1, January 1995 (1995-01), pages 133-139, XP001057706 ISSN: 0963-6897
- RICHARD G ET AL: "TRANSPLANTATION VON NETZHAUT-BESTANDTEILEN RETINAL TRANSPLANTATION - A REVIEW" KLINISCHE MONATSBLAETTER FUER AUGENHEILKUNDE, FERDINAND ENKE VERLAG, STUTTGART, DE, vol. 206, no. 2, February 1995 (1995-02), pages 71-77, XP001057997 ISSN: 0023-2165
- KLEPPNER S R ET AL: "TRANSPLANTED HUMAN NEURONS DERIVED FROM A TERATOCARCINOMA CELL LINE(NTERA-2) MATURE, INTEGRATE, AND SURVIVE FOR OVER 1 YEAR IN THE NUDE MOUSE BRAIN" JOURNAL OF COMPARATIVE NEUROLOGY, XX, XX, vol. 357, 1995, pages 618-632, XP002915301
- TROJANOWSKI J Q ET AL: "NEURONS DERIVED FROM A HUMAN TERATOCARCINOMA CELL LINE ESTABLISH MOLECULAR AND STRUCTURAL POLARITY FOLLOWING TRANSPLANTATION INTO THE RODENT BRAIN" EXPERIMENTAL NEUROLOGY, SAN DIEGO, CA, US, vol. 122, no. 2, August 1993 (1993-08), pages 283-294, XP001057992
- CERRO DEL M ET AL: "NEURAL RETINAL TRANSPLANTATION INTO TWELVE R.P. PATIENTS" INVESTIGATIVE OPHTHALMOLOGY & VISUAL SCIENCE, ASSOCIATION FOR RESEARCH IN VISION AND, US, vol. 38, no. 4, 15 March 1997 (1997-03-15), page S261 XP002911861 ISSN: 0146-0404
- GELLRICH N C ET AL: "INFLUENCE OF FETAL BRAIN GRAFTS ON AXOTIMIZED RETINAL GANGLION CELLS" INTERNATIONAL JOURNAL OF ORAL AND MAXILLOFACIAL SURGERY, COPENHAGEN, DK, vol. 23, no. 6, PART 2, December 1994 (1994-12), pages 403-405, XP001057519 ISSN: 0901-5027
- SHINODA ET AL.: "DIFFERENTIAL IMMUNE RESPONSES TO FETAL INTRACAMERAL SPINAL CORD AND CORTEX CEREBRI GRAFTS" EXPERIMENTAL BRAIN RESEARCH, vol. 110, 1996, pages 223-234, XP001056161
- HONJO H ET AL: "ESTROGEN AS A GROWTH FACTOR TO CENTRAL NERVOUS CELLS. ESTROGEN TREATMENT PROMOTES DEVELOPMENT OF ACETYLCHOLINESTERASE-POSITIVE BASAL FOREBRAIN NEURONS TRANSPLANTED IN THE ANTERIOR EYE CHAMBER" JOURNAL OF STEROID BIOCHEMISTRY AND MOLECULAR BIOLOGY, ELSEVIER SCIENCE LTD., OXFORD, GB, vol. 41, no. 3/8, 1992, pages 633-635, XP002911863 ISSN: 0960-0760
- INVESTIGATIVE OPHTHALMOLOGY & VISUAL SCIENCE, 15 March 1997, Vol. 38, No. 4, DEL CERRO et al., "Neural Retinal Transplantation into Twelve R.P. Patients" page S261, Abstract No. 1204-6:15, XP002911861
- EXPERIMENTAL NEUROLOGY, July 1996, Vol. 140, DILORETO et al., "Cyclosporine Treatment Promotes Survival of Human Fetal Neural Retina Transplanted to the Subretinal Space of the Light-Damaged Fischer 344 Rat", pages 37-42, XP002911862
- STEROID BIOCHEM. MOLEC. BIOL., 1992, Vol. 41, No. 3-8, HONJO et al., "Estrogen as a Growth Factor to Central Nervous Cells", pages 633-635, XP002911863
- EXPERIMENTAL BRAIN RESEARCH, June 1980, Vol. 39, No. 3, OLSON et al., "Conditions for Adrenergic Hyperinnervation in Hippocampus: I. Histochemical Evidence from Intraocular Double Grafts", pages 277-288, XP002911864

## Description

### Field of Use

The present invention is in the field of human transplantation and more particularly in the field of intraocular and intracranial transplantation of specially treated human cells which reestablish neuronal connections, which neurons having been damaged by a variety of causes.

### Background Information

Blindness is a leading cause of disability in the world. It has many causes and no effective treatment, except attempting to arrest the progressive vision loss, which is not possible in many cases. In recent years, neuroscientists have been performing various animal transplants to determine if blindness can be treated effectively. Aramant and Seiler reported that embryonal retinal transplants from either rats or humans to adult rats formed synapses with host retinal fibers, an essential step in replacing retinal nerve cells. First, the rats received retinal lesions (a small incision through the sclera, choroid and retina which was sutured). Then broken-up cell aggregates of embryonic retina were injected into the vitreous to cover the lesion site. The eyes were observed 3 to 11 months after transplant. Dye-labelled transplant cells exhibited fiber outgrowth into the host retina; and host fibers grew into the transplants. (Experimental Neurology 133:244-55, 1995).

Implants have also been tried in RCS rats which have early-onset, hereditary blindness due to photoreceptor degeneration. The retinal pigment epithelium (RPE) cannot phagocytize the shed rod outer segments, so debris accumulates and causes the photoreceptor cells to degenerate. Little et al. reported that transplantation of fragments of human fetal RPE to the subretinal space "rescued" the photoreceptors. After four weeks, the RPE cells were visible overlying the retina, and the underlying nuclear layer was three to four nuclear profiles thick, in contrast to the untreated area where nuclear profiles were all but absent, indicating loss of cells and vision. (Invest. Ophthalmol. Vis Sci. 37:204-11, 1996).

Human fetal neural retina cells were also transplanted into the sub-retinal space in cyclosporine immunosuppressed rats, DiLoreto et al, Experimental Neurology 1996, 140 37-42.

Glaucoma is an important cause of blindness and occurs in 1-2% of individuals over the age of 60. Often the disease is asymptomatic, as the patient painlessly and gradually loses vision. Before a diagnosis is made, the patient may have lost half of the one million optic nerve fibers in one eye. Today, intervention is focused on early detection, which depends on a routine eye examination which includes intraocular pressure measurement (tonometry), funduscopy with attention to the optic disc appearance, and visual field testing.

Glaucoma is often asymmetric. Vertical disparity in one or both eyes is an early sign of glaucoma. The finding of asymmetry of the cup-disc ratio implies glaucoma. Early in the disease, visual field loss may include nonspecific constriction and small paracentral scotomas. Eventually, the arcuate nerve fiber bundle defects develop with a characteristic nasal step: The arcuate bundle defect extends to the nasal horizontal raphe to form a step-like configuration on kinetic visual field testing. The papillomacular bundle is spared until late in the disease. Intraocular pressure reflects the balance between the production and outflow of aqueous humor. Treatment involves controlling intraocular pressure with topical agents including cholinergic (pilocarpine, carbachol, echothiophate) or adrenergic agonists (epinephrine dipivefrin) or antagonists such as β-adrenergic blockers including timolol, levobunalol and betaxolol. If topical agents do not reduce the intraocular pressure sufficiently, systemic carbonic anhydrase inhibitors such as acetazolamide or methazolamine are added. If medical therapy fails, surgery is tried, such as laser trabeculoplasty or filtration surgery, to improve aqueous outflow.

Retinal degenerative diseases are a growing problem for which there is no cure. They can be broadly categorized by the anatomical location of the abnormalities. Degeneration in diseases known as retinitis pigmentosa (RP) typically proceeds in a peripheral to central gradient. Macular degeneration, in contrast, affects the central region of the retina. RP affects about 1 in 3,000 across all ethnic groups. Age-related macular degeneration (AMD), the predominant form of macular degeneration, affects six million people in the United States alone, approximately one in every 40 people. In both RP and macular degeneration, there is progressive loss of photoreceptors. Many groups have evaluated the possibility of transplanting slices of retina, photoreceptors and/or retinal pigment cells (see above). While promising, there are a number of challenges, including difficulties isolating and obtaining single cell suspensions that differentiate appropriately, identifying the cells after transplant and establishing whether the neuronal cells form synaptic contacts.

Compression of the optic nerve causes insidious progressive visual and field loss. The disc may be normal, swollen or atrophic. Intrinsic tumors which may compress the optic nerve include optic nerve sheath meningioma and glioma. In Graves' ophthalmopathy, optic neuropathy is due to compression of the nerve in the orbital apex by the enlarged extraocular muscles. Benign or malignant orbital tumors, metastatic lesions, tumors arising from the adjacent paranasal sinuses and middle cranial fossa and giant pituitary adenomas can each lead to compressive optic neuropathy.

Vision may be lost if papilledema is not promptly treated. Papilledema is swelling of the optic nerve head due to increased intracranial pressure. It is usually bilateral and occurs with brain tumors and abscesses, cerebral trauma and hemorrhage, meningitis, arachnoidal adhesions, pseudotumor cerebri, cavernous sinus thrombosis, dural sinus thrombosis, encephalitis, space-occupying brain lesions, severe hypertensive disease and pulmonary emphysema.

Vision also can be lost due to higher visual pathway lesions. The retinal nerves gather into the optic nerve, which may be inpinged on in its pathway to the optic chiasma. At the optic chiasma the optic nerve fibers from the medial halves of both retina cross to the opposite side before connecting to the occipital visual cortex. Lesions at the optic chiasma tend to cause bilateral vision loss. Lesions at the visual cortex cause vision loss in the retinas on the same side as the cortical lesion. Thus, vision is vulnerable to a number of different pathologies in a variety of intracranial locations.

Even if the patient obtains appropriate treatment, treatment today is limited to stopping further progression of vision loss, not improving vision. Because the patient may have already lost so much peripheral vision that be is effectively blind, the patient may not be permitted to drive, which may cause loss of job and independence, important deterioration in productivity and quality of life. A method of restoring at least some of the vision to enable the patient to return to work and other activities is sorely needed.

Neural transplantation has been tried as a therapy in several animal models of Parkinson's disease and other neurodegenerative disorders (Bjorklund and Stenevi, Brain Res. **177**:555-60, 1979; Sanberg et al., CELL TRANSPLANTATION FOR HUNTINGTON'S DISEASE, R.G. Landes Company, Austin TX, 1994, p 124). This experimental treatment has been applied clinically in Parkinson's disease (PD) patients with favorable results (Lindvall et al., Science **247**:574-77, 1990; Kordower et al., New Engl. J. Med. **332**:1118-24, 1995; Freeman et al., Ann. Neurol. **38**:379-88, 1995).

However, logistical and ethical problems hinder widespread use of human fetal tissue for human neural transplantation (Borlongan et al., Neurolog. Res. 18:297-304, 1996b). Alternative graft sources have been explored, such as encapsulated cells and genetically engineered cells (Emerich et al., 1996, *ibid*.; Kawaja et al., J. Neurosci. **12**:2849-64, 1992). However, there is a need to develop cell lines that generate large numbers of differentiated or post-mitotic cells for human transplantation therapies (Mantione et al., Brain Res. Bull. **671**:33-337, 1995).

Recently treated cultured human neuronal cells (NT2 neuron cells derived from an embryonal cell line isolated from a human teratocarcinoma (Ntera2 or NT-2/D1™ cells) were transplanted into the rodent brain (Kleppner et al., J. Comp. Neurol. **357**:618-32, 1995; Miyazono et al., Brain Pathol. 4:575, 1994; Trojanowski et al., Exp. Neurol. **122**:283-94, 1993). After retinoic acid treatment, NT-2/D1 cells differentiated into post-mitotic neuron-like (hNT neuron) cells (Pleasure et al., J. Neurosci. Res. **35**:585-602, 1992). In vivo studies indicate that transplanted hNT neuron cells can survive, mature and integrate into host brain (Kleppner et al., 1995, *ibid*.; Mantione et al., 1995, *ibid.;* Trojanowski et al., 1993, *ibid.*). Transplanted subjects have been observed for more than one year, during which none of the transplanted hNT neurons have reverted to a neoplastic state.

These features of human pluripotent stem cells, human neuronal stem cells and hNT cells, coupled with the localized lesion of certain types of vision losses, provided the basis for investigating the effects of hNT neuronal cell transplantation on vision loss.

### Summary of Disclosure

Vision loss in a mammal can be treated by administering an effective amount of human pluripotent stem cells or human neuronal stem cells, such as hNT neurons, after the vision loss is diagnosed.

In another embodiment, vision loss in a mammal is caused by glaucoma or a compression injury of the optic nerves.

In another embodiment, vision loss is treated by injecting hNT neurons into the eye. Alternately, or additionally, hNT neurons are injected into the visual cortex. In yet another embodiment, the mammal is treated with an immunosuppressant drug.

In yet another embodiment, vision loss is due to optic nerve sheath meningioma and glioma, Graves' ophthalmopathy, benign or malignant orbital tumors, metastatic lesions, tumors arising from the adjacent paranasal sinuses or middle cranial fossa, giant pituitary adenomas, brain tumors or abscesses, cerebral trauma or hemorrhage, meningitis, arachnoidal adhesions, pseudotumor cerebri, cavernous sinus thrombosis, dural sinus thrombosis, encephalitis, space-occupying brain lesions, severe hypertensive disease, pulmonary emphysema, or retinal degeneration.

### Description of Drawings

FIG. 1 is a schematic showing a method of hNT injection into the eye near the optic nerve.
FIG. 2A is a schematic showing the locations of the cryostat sections collected at the level of the optic disk, as well as 0.5 mm dorsal and ventral to the optic disk. FIG. 2B is a schematic showing the horizontal section of the retina collected in A. Three zones (nasal, center and temporal) were sampled quantitatively for hNT survival, laminar distribution and spatial distribution.
In FIGS. 3A to 5D, locations of the arrowheads in the fluorescent micrographs are identical to locations in phase contrast micrographs.

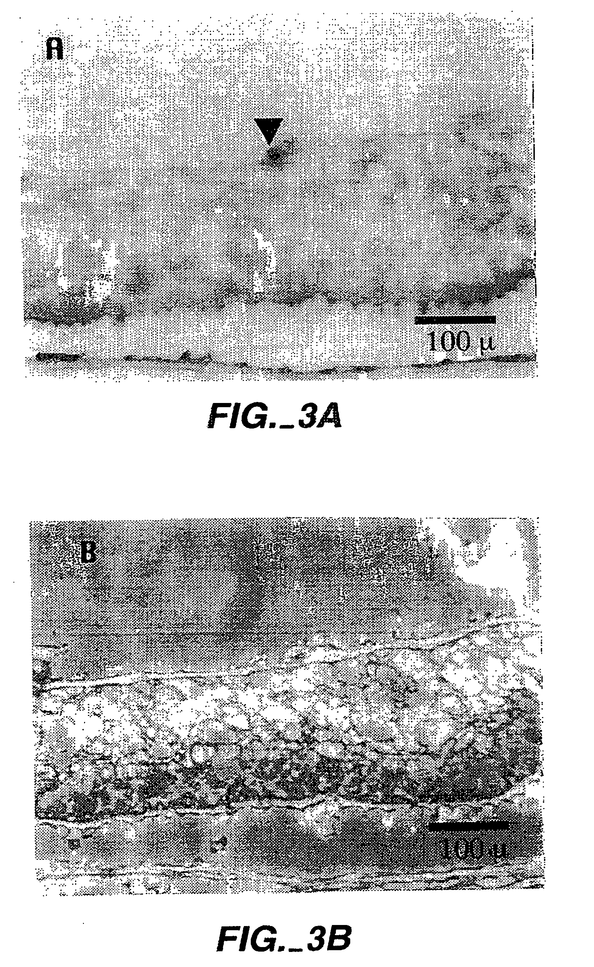

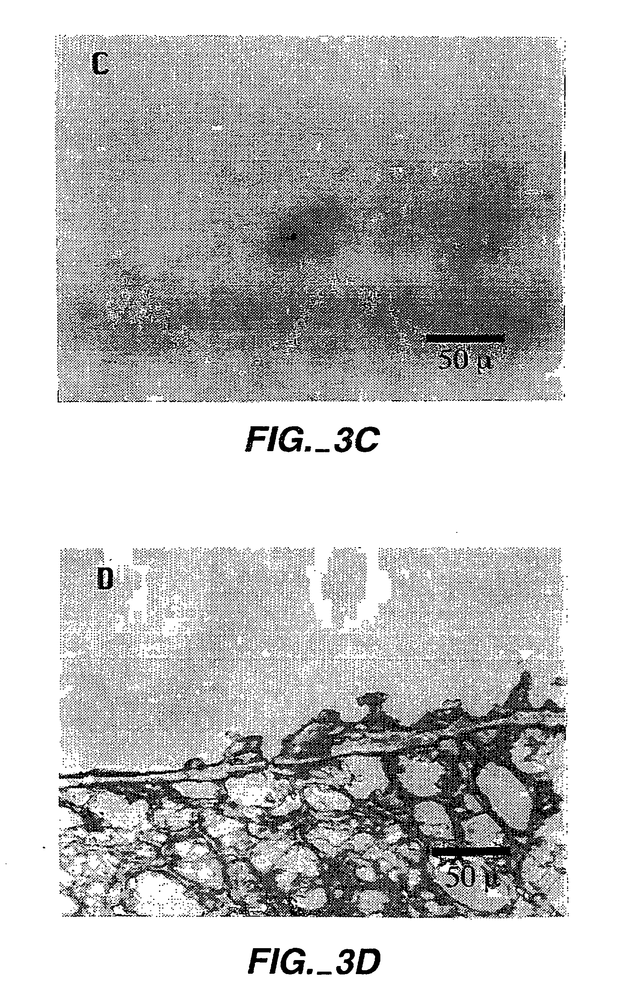

FIG 3A is a fluorescent micrograph showing fluorescent dye 1,1'-dioctadecyl-3,3,3',3'-tetramethylindocarbocyanine perchlorate (DiI)-labeled 3-day hNT cells with 20X magnification. FIG.3B is a phase-contrast-micrograph of the same field as in FIG. 3A and same magnification. FIG. 3C is a fluorescent micrograph showing fluorescent dye 1,1'-dioctadecyl-3,3,3',3'-tetramethylindocarbocyanine perchlorate (DiI)-labeled 3-day hNT cells with 40X magnification. FIG.3D is a phase-contrast-micrograph of the same field as in FIG. 3C and same magnification.

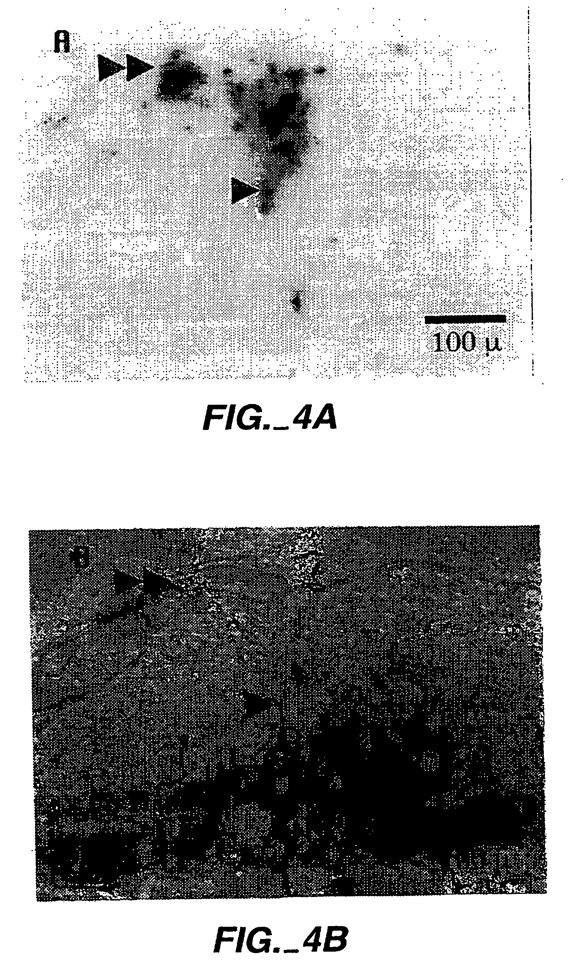

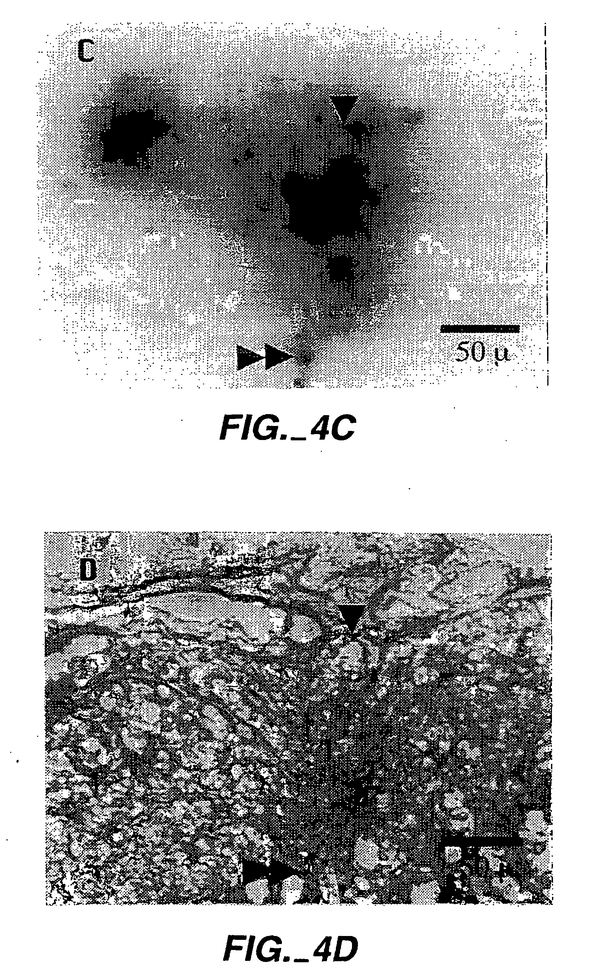

FIG 4A is a fluorescent micrograph showing fluorescent dye 1,1'-dioctadecyl-3,3,3',3'-tetramethylindocarbocyanine perchlorate (DiI)-labeled 14-day hNT cells with 20X magnification. FIG.4B is a phase-contrast-micrograph of the same field as in FIG. 4A and same magnification. FIG. 4C is a fluorescent micrograph showing fluorescent dye 1,1'-dioctadecyl-3,3,3',3'-tetramethylindocarbocyanine perchlorate (DiI)-labeled 14-day hNT cells with 40X magnification. FIG.4D is a phase-contrast-micrograph of the same field as in FIG. 4C and same magnification.

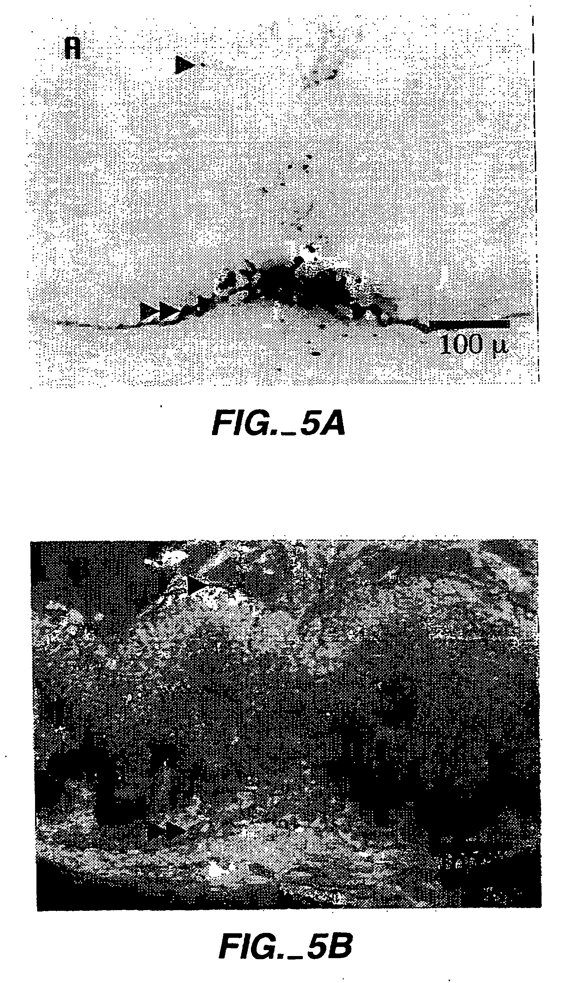

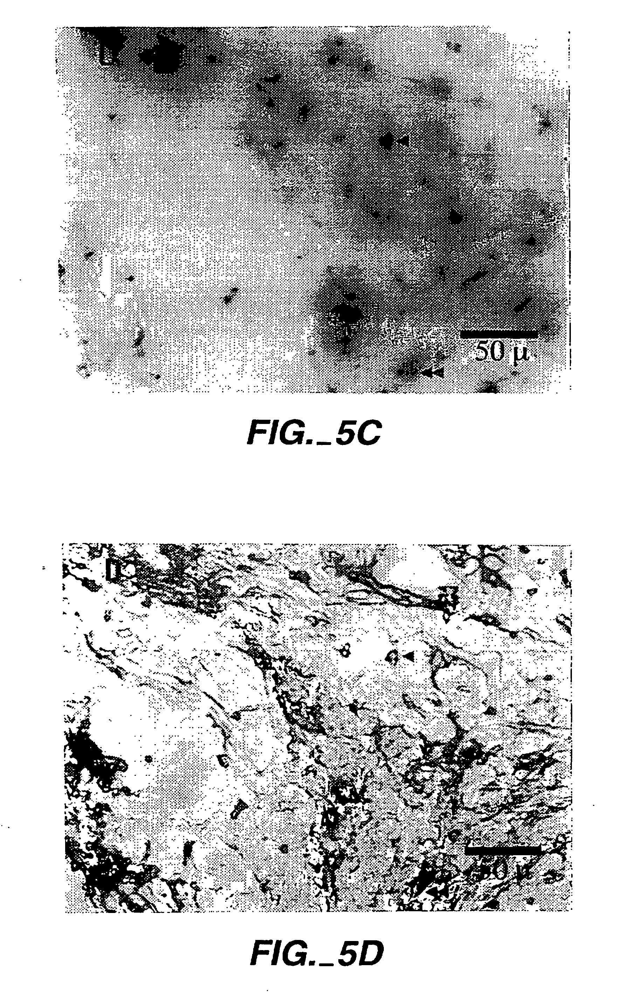

FIG 5A is a fluorescent micrograph showing fluorescent dye 1,1'-dioctadecyl-3,3,3',3'-tetramethylindocarbocyanine perchlorate (DiI)-labeled 28-day hNT cells with 20X magnification. FIG.5B is a phase-contrast-micrograph of the same field as in FIG. 5A and same magnification. FIG. 5C is a fluorescent micrograph showing fluorescent dye 1,1'-dioctadecyl-3,3,3',3'-tetramethylindocarbocyanine perchlorate (DiI)-labeled 28-day hNT cells with 40X magnification. FIG.5D is a phase-contrast-micrograph of the same field as in FIG. 5C and same magnification.

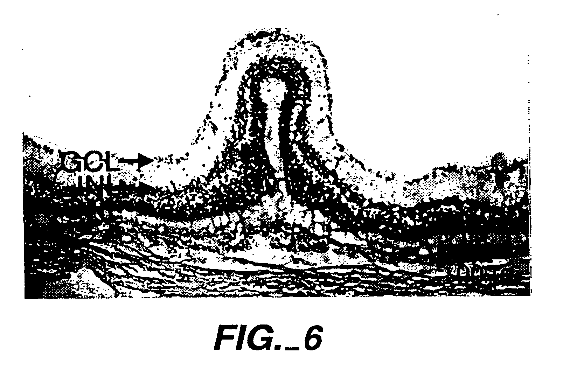

FIG. 6 shows exudative detachment of the retina following DiI-labeled hNT transplant in the 14-day survival group.

### Detailed Description

The present invention arose out of an observation relating to the positive results reported in U.S. Patent Application Serial No. 08/797,952, filed on February 12, 1997, and entitled "TROPHIC FACTOR FROM hNT-NEURON™ HUMAN NEURONAL CELLS TO TREAT NEUROLOGIC AILMENTS" and invented by Cesario Borlongan, Paul Sanberg and Gary Snable. Transplanted hNT cells grew and took hold regardless of which brain tissue they encountered. The transplanted hNT cells maintained their mature neuronal status during the six-month study. Because it is well known that new nerves will grow down the same pathways which accommodated the older nerves, I conceived a method for treating blindness, such as is caused by glaucoma or other forms of optic nerve compression. Also, because the hNT neurons successfully replaced cranial nerves which are believed to die from excitotoxicity (GABA and glutamate-sensitive cells), I believe that the hNT neurons also will adequately replace retinal and optic nerves which also have GABA and glutamate receptors.

Previously, the biological effects of transplanting cultured human neurons (hNT neurons) derived from a well characterized human embryonal carcinoma cell line into the brains of rats subjected to stroke-like ischemic injury were investigated. The rat stroke model is characterized by transient, focal cerebral ischemia following embolic occlusion of the middle cerebral artery. At one month and extending throughout the 6-month post-transplantation test period, ischemic rats transplanted with hNT neurons and treated with immunosuppression displayed a significant improvement in a passive avoidance learning and memory task. Their asymmetrical motor behavior also normalized more than that of ischemic rats receiving rat fetal cerebellar cell transplantation or vehicle infusion. While ischemic rats given rat fetal striatal cells also exhibited significant behavioral improvement, hNT transplanted animals showed more robust recovery at one month after transplantation. Ischemic animals receiving hNT neurons without immunosuppression showed significant behavioral recovery at one month after transplantation. Nevertheless, thereafter, they reverted to the post-ischemia levels that preceded transplantation. With monoclonal and polyclonal antibodies to the low-molecular-weight neurofilament protein as well as to human neural cell adhesion molecules, surviving hNT neurons were detected at 3 and 6 months after transplantation in immunosuppressed animals that displayed significant behavioral improvement. Thus, transplanted hNT neurons appeared to promote functional recovery. This supports the utility of hNT neurons as an alternative graft source for the treatment of transient, focal cerebral ischemia and possibly other neurodegenerative disorders. Definitions:
"Impaired vision" or "vision loss" refers to the clinically observable signs and symptoms of loss of vision due to loss of central nervous system neurons. Impaired vision as used herein does not encompass near- or far-sightedness, presbyopic or cataracts which have other causes. Loss of vision is detectable by a variety of diagnostic tests and clinical observations which are well known in the medical profession. Such tests include visual field testing. In classical glaucoma, peripheral vision is lost. It may be noted initially by auto-side-swiping accidents or by testing by a health care provider.

Examples of causes of loss of vision because of loss of central nervous system neurons include but are not limited to glaucoma, optic nerve sheath meningioma and glioma, Graves' ophthalmopathy, benign or malignant orbital tumors, metastatic lesions, tumors arising from the adjacent paranasal sinuses and middle cranial fossa, giant pituitary adenomas, brain tumors and abscesses, cerebral trauma and hemorrhage, meningitis, arachnoidal adhesions, pseudotumor cerebri, cavernous sinus thrombosis, dural sinus thrombosis, encephalitis, space-occupying brain lesions, severe hypertensive disease and pulmonary emphysema.

"Beneficial effect" is an observable improvement over the baseline clinically observable signs and symptoms of vision loss. For example, a beneficial effect could include improvements in peripheral vision, if that were lost.

"Mammal" includes humans and other mammals who would reasonably benefit from treatment of stroke, including pets like dogs, cats and horses.

"NT-2/D1™ precursor cells" as used herein refers to a special cell line available from Stratagene (Palo Alto, CA). This cell line has been developed from a previously described human teratocarcinoma cell line (termed Ntera2/clone DI or NT2 cells) (Andrews et al. Lab. Invest. 50:147-162, 1981). These cells are precursors for hNT-Neuron™ human neuronal cells. NT2 cells are unique among other teratocarcinoma cell lines because these cells act like progenitor cells whose progeny are restricted to the neuronal lineage (Andrews, *ibid*.)

"hNT Neurons or hNT cells" as used herein refers to the special neuronal cell line disclosed in U.S. Patent No. 5,175,103 to Lee et al. Briefly, NT-2/D1 precursor cells are induced to differentiate into neurons by administration of 10 µM retinoic acid which is replenished twice weekly for 5 weeks, after which the cells are replated three times with special manipulations to become more than 99% pure hNT neurons. These are the cells which are used in the subsequent experiments. Alternately, for human use, there is a cell line manufactured without antibiotics (used in the research grade hNT-Neuron cells) and under good manufacturing practices (GMP) which is termed LBS NEURONS™ human neuronal cells.

"Immunosuppressant" as used herein is a substance which prevents, attenuates and/or treats the host versus graft rejection which can occur when an allogenically different cell line or tissue is transplanted into a host. Examples of immunosuppressants include but are not limited to cyclosporine A, cyclophosphamide, prednisone and tacrolimus.

### EXAMPLES

### Example 1

Adult Male Wistar rats (350-450g; Harlan, Indianapolis IN) were housed in cages with food and water ad libitum. All rats were administered tacrolimus intramuscularly daily, beginning seven days prior to hNT injection. The rats were divided into three experimental groups: 3 days survival, 14-day survival and 28-day survival. All rats were weighed before hNT injection and weekly thereafter. None lost a significant amount of weight.

Frozen PF-hNT cells (Layton Bioscience, Inc., Atherton, CA) were prepared according to manufacturer's protocol as follows: liquid nitrogen-frozen cells (about 10 million per ml) were thawed rapidly in a 37°C water bath with occasional swirling. Cells were suspended in 12 ml DMEM, centrifuged 300 g for 15 minutes at 4°C, decanted and resuspended in 12 ml DMEM containing 2.7µM DiI. Cells were incubated on ice for 15 min and centrifuged 300 g for 15 minutes at 4°C. DiI-containing supernatant was discarded, the pellet rinsed superficially with DMEM and resuspended in 1 ml DMEM. Cell density and viability were determined with a hemacytometer by 0.4% trypan blue exclusion under phase contrast and adjusted for a final density of 30,000 cells/µl.

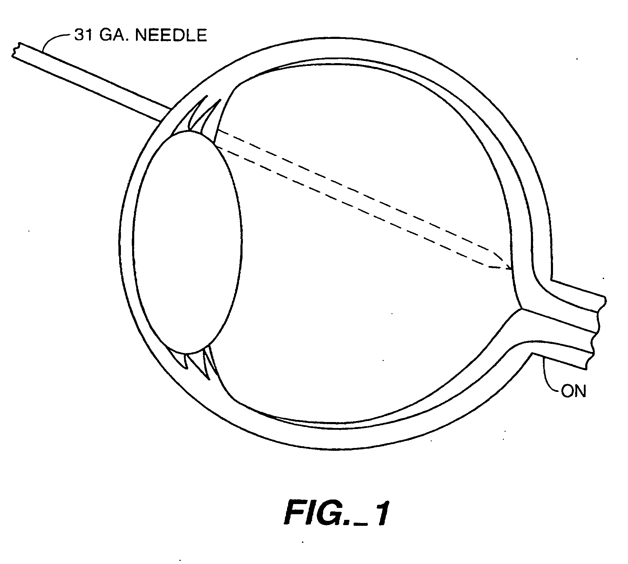

Under halothane anesthesia (2.5-3.0% in 95% O₂ / 5% CO₂), animals were placed in a stereotaxic apparatus. Their eyelids were held open with forceps, while the globe was held in place using a forceps. A 10µl Hamilton syringe was used to gently inject 1 µl of the hNT suspension epiretinally via a 31-gauge needle which penetrated the cornea and vitreous cavity, such that the tip of the needle rested near the optic disk at the vitreous-retina interface (FIG. 1). Only one eye per rat was transplanted.

After 3, 14 or 28 days, the rats were deeply anesthetized and decapitated. Their eyes were removed and the cornea immediately slit and placed in 4% paraformaldehyde at 4°C overnight. After fixation, the eyes were embedded in Tissue-Freezing Medium (Triangle Biomedical Sciences), frozen and stored at -70°C until use. Cryostat section (14µm) were cut horizontally at -27°C and then mounted onto gelatin-coated slides. The coordinates of each section were recorded for three-dimensional reconstruction of the data.

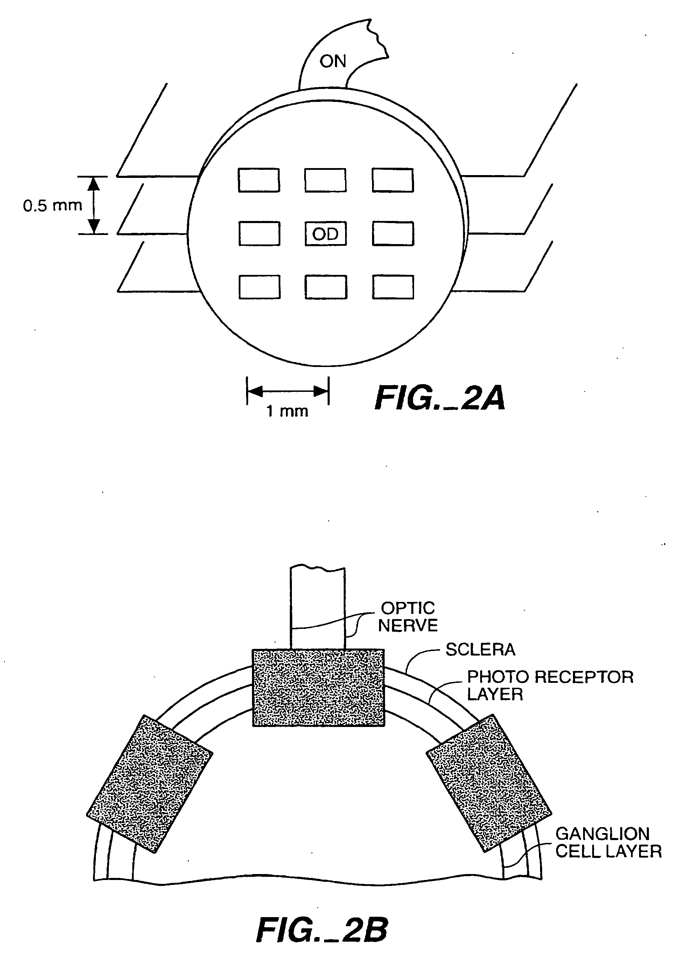

The sections were examined under a microscope with epifluorescence illumination. A fluorescein filter block and uninjected eyes were used to identify autofluorescent cells. Cells were counted as follows at nine points in each eye: Counting was performed at the optic disk in the horizontal section bisecting the optic disk and at points 1 mm lateral to it. Analogous points 500 µm dorsal and ventral to the first section were quantitated similarly (FIG. 2).

In each experimental group, hNT cells with DiI could be identified easily by fluorescence microscopy (FIGS. 3, 4 and 5). Labeled hNT cells appeared bright under DiI but not fluorescein filter. In contrast, there was no fluorescence in the eyes in which no DiI-labeled cells were injected, except for autofluorescent tissue (ciliary body, for instance), which was brightly fluorescent under fluorescein filter as DiI filter. The fluorescent images (FIGS. 3A, 3C, 4A, 4C, 5A and 5C) were digitally inverted so that the fluorescent cells which appear bright under fluorescence microscopy are printed as dark. At 3 days survival, hNT cells were found primarily in the vitreous and at the vitreous-retinal interface (Table 1). Most hNT cells were found in the vitreous, sometimes close to the retinal surface, but most often in vitreous distant from the retina. Rarely, hNT cells were found superficially attached to the ganglion cell layer of retina and within the retina, mostly in the ganglion cells layer (FIG. 2A-3D). The hNT cells were generally round, and retinal appearance was well preserved.

At 14 days survival, most hNT cells were in the vitreous (Table 1). Those in the retina were either superficially attached in the ganglion cell layer; few had

**Table 1.**

| **Laminar distribution of hNT cells in retina** (average value from nine points sampled) | | | |
|---|---|---|---|
| | **3 day** | **14 day** | **28 day** |
| Intra-retinal | 0% | 10% | 17% |
| Epiretinal | 33% | 43% | 31% |
| Vitreous | 67% | 47% | 52% |
| | | | |
| All layers | 100% | 100% | 100% |

| | **3 day** (n=4) | | |
|---|---|---|---|
| | Nasal | Center | Temporal |
| Dorsal | 11% | 8% | 0% |
| Center | 7% | 41 % | 6 % |
| Ventral | 10% | 11% | 6% |
| | | | |
| hNT density, #/mm | 34±19 | | |

| | **14 day** (n=5) | | |
|---|---|---|---|
| | Nasal | Center | Temporal |
| Dorsal | 9% | 8% | 8% |
| Center | 13% | 15% | 9% |
| Ventral | 11% | 17% | 11% |
| | | | |
| hNT density, #/mm | 275±103 | | |

| | **28 day** (n=5) | | |
|---|---|---|---|
| | Nasal | Center | Temporal |
| Dorsal | 6% | 6% | 7% |
| Center | 17% | 11% | 4% |
| Ventral | 31% | 11% | 6% |
| | | | |
| hNT density, #/mm | 135±37 | | |

**Table 2. Topographic distribution of hNT cells in rat retina as a function of survival time.** The numbers of hNT cells found in nine different regions of the retina were determined in all eyes. Values given are percentages of the total number of hNT cells for each survival time. At three days survival, there was a markedly higher concentration of the cells in the core ('center-center'), which was not observed at 14 and 28 days survival. penetrated deeper into the retina. hNT cells only integrated into the retina at damaged locations. Most cells were round, but some had processes.

At 28 days survival, hNT cells were still found predominantly in the vitreous, but had attached to the ganglion cell layer and penetrated as deeply as the photoreceptor layer (Table 1, FIGS. 5A and 5B). The hNT cells in the retina or vitreous were multipolar and some cells had identifiable nucleus and processes (FIGS. 5C and 5D).

Table 1 shows the distribution of surviving hNT cells in the different layers. Except for the 3 day survival group, hNT cells were found in each layer. The 3 day group had no cells deeper than the ganglion cell layer. In each group, surviving hNT cells were found in the vitreous.

Table 2 shows the topographic distribution and total number of hNT cells as a function of survival time. The injection was targeted at the optic disk, but hNT cells were found far from the optic disk at all survival times; therefore, strict administration at the optic disk is not essential. The cells migrate from the injection point to other locations. Except for the dorso-temporal region at 3 days, cells were found in all regions. hNT cells were concentrated near the optic disk at 3 days, more than at later survival time, i.e., 41% found in this sector at 3 days vs. 15% and 11% at 14 and 28 days, respectively.

Based on the counts of hNT cells in selected section from each retina, densities were established and averaged. The average density was multiplied by the total surface area of the retina, reconstructed from sectioning coordinates, to yield estimated totals of 1.7 X 10³, 13.2 X 10³ and 7.1 X 10³ cells at 3, 14 and 28 days survival, respectively. These values only reflect those cells found near the retina and exclude cells found in the center of the vitreous, which made up the majority of cells at 3 days survival. Nevertheless, the estimates suggest that of the 20,000 hNT cells injected into each eye, 5.7%, 44% and 24% were found in the regions sampled at days 3, 14 and 28. At 3 days, the majority of cells likely were still in the vitreous, which was not sampled. The decrease in cell numbers from day 14 to 28 may reflect cell death or more lateral migration (another unsampled area).

As mentioned about, the hNT cells apparently migrated in the eye. At 3 days no cells were found deeper than the superficial ganglion cell layer, but by 14 days cells counted in the retina increased and in the vitreous decreased. After an additional 14 days, hNT cells increased in the deep retina from 10% to 17%. Furthermore, in those 14 days, cells in the retina appeared to shift from predominance in the ganglion cell layer to the photoreceptor layer and took on the appearance of funnel-like inclusions of hNT cells (FIGS. 4A-5B). Others have shown that allotypic grafts consisting of cell suspensions survive, differentiate, and integrate in the rat and check retina and chick optic tectum, which is probably happening in this study. Further evidence of that is the changing hNT morphology from round at 3 days to multipolar, with identifiable processes at 14 and 28 days.

In four of five injected eyes in the 28 day survival group, some sort of retinal detachment (FIG. 6) was observed at selective loci. Similar detachments also were seen in two of five at 14 days and one of four at 3 days. Typically, such areas of detachment were very small compared to the total retinal surface, and the retinal layers were still intact. Areas did not show any preferential colonization by DiI-labeled hNT cells. Such detachments have been reported after intraocular injections, the probable cause.

In conclusion, it was proved that hNT cells survived at least 28 days and apparently migrate into the retina, where they differentiate and may well integrate into the retinal circuitry.

### Example 2

In this study of hNT cell incorporation in mouse retina, commercially available, LacZ-expressing hNT cells and hNT cells transfected with green fluorescent protein (GFP). While lacZ has been used successfully in the subretinal space, it is not detectable until tissue fixation and histochemical reaction. GFP can be detected in vivo and would allow periodic ophthalmic examination of the hNT cell progress; it can also be detected with a fluorescent microscope.

Rodents with spontaneous mutations and those that have been genetically engineered to contain mutations identified in humans with retinal degeneration are used to further evaluate the ability of hNT cells to undergo site specific terminal differentiation. Different strains of rodents with retinal degeneration are used, as they differ dramatically in their rate of retinal degeneration (ranging from 2 weeks to 6 months to the time of total photoreceptor loss). The specific strains include wild-type mice, *rd*/*rd* mice, *rds*/*rds* mice, mutant rhodopsin transgenic mice, wild-type rats and mutant rhodopsin transgenic rats.

In all cases, hNT neurons, labeled with a reporter gene, are injected into the subretinal space. The dose obviously varies with the rodent size. Subretinal injection is performed using surgical techniques developed by Bennett et al. (Invest Ophthalm & Vis Sci 35:2535-42, 1994), a technique that does not disrupt synaptic and intraretinal neural connections, and whose damage is largely reversible. Location of the transplanted cells is evaluated with ophthalmoscopy as a function of time after transplantation (evaluating GFP-mediated fluorescence). Cohorts of animals are sacrificed at various intervals following transplantation, and tissue is assess for presence and status of hNT cells. Markers specific for RPE cells, photoreceptors, amacrine cells, bipolar cells, Muller cells and ganglion cells are used to assess the degree of differentiation of hNT cells.

The ability of the hNT cells to differentiate is also evaluated functionally. electrophysiological measurements of photoreceptor function (ERGs) are performed to provide quantitative assessment of rescue of visual function. By manipulating the type of stimulus to elicit the ERG, rod and cone photoreceptor functions can be separated. ERGs are sensitive indicators of photoreceptor degeneration and have been used to assess the state of retinal function. Retinal function of the treated portion of the retina is compared with that of the untreated or sham-injected second eye. Localized measures of photoreceptor integrity are optionally performed with imaging fundus reflectometry.

### Example 3

A study is disclosed for treatment of vision loss due to glaucoma. Beagles which congenitally acquire glaucoma at about 12 to 18 months are maintained until they show significant signs and symptoms of loss of vision. The mature animals are divided into groups for dosing. Dosing is determined based on the number of ocular nerves which may need to be replaced. It has been estimated that humans lose as many as 5X10⁵ nerve cells in glaucoma. Dosing is vehicle alone (control), 4X10⁴, 8X10⁴ and 12X10⁴ cells per injection volume of 3 µl.

The ocular entry procedure is similar to procedures used by ophthalmologists to gain access to the retina and aqueous humor. Entry with a relatively narrow-gauge needle is made at a small sclerotomy incision and the tip of the needle is positioned in the retinal space. The injection volume is delivered slowly, over at least three minutes, to avoid retinal detachment. The needle is left in place for an additional five minutes to avoid letting nerve cells migrate away from the retina.

Alternately or additionally, each animal can be injected in the visual cortex at the dorsal surface of the brain. For peripheral vision losses, the visual cortex area is relatively small and can be located stereotaxically.

The test animals are observed for six months to two years to record vision improvements. At the end of the study, autopsies are performed. Special precautions are taken to assure that the eye and brain tissue are properly preserved to permit analysis of the slides for growth of neurons. Anti-hNT antibodies are applied to the slides to distinguish the mature hNT neurons from the animals' own neurons.

The foregoing description and examples are intended only to illustrate, not limit, the disclosed invention.

## Claims

1. Use of stem cells hNT neurons in the manufacture of a medicament for the treatment of vision loss or blindness in a mammal.

2. Use of hNT neurons according to claim 1 wherein the vision loss is caused by glaucoma.

3. Use of hNT neurons according to claim 1 or 2 wherein hNT neurons are for injection into the eye.

4. Use of hNT neurons according to claim 3 wherein the hNT neurons are additionally for injection into the visual cortex.

5. Use of hNT neurons according to claim 1 wherein hNT neurons are for injection into the visual cortex.

6. Use of hNT neurons according to any one of the preceding claims wherein the mammal also is to be treated with an immunosuppressant drug.

7. Use of hNT neurons according to any one of claims 1 or 3 to 6 wherein the vision loss is caused by optic nerve sheath meningioma and glioma, Graves' ophthalmopathy, benign or malignant orbital tumors, metastatic lesions, tumors arising from the adjacent paranasal sinuses or middle cranial fossa, giant pituitary adenomas, brain tumors or abscesses, cerebral trauma or haemorrhage, meningitis, arachnoidal adhesions, pseudotumor cerebri, cavernous sinus thrombosis, dural sinus thrombosis, encephalitis, space-occupying brain lesions, severe hypertensive disease or pulmonary emphysema.

8. Use of hNT neurons according to claim 1 wherein the vision loss is due to retinal degeneration.

9. Use of hNT neurons according to claim 8 wherein the degeneration is age-related macular degeneration.

## Patentansprüche

1. Verwendung von Stammzellen-hNT-Neuronen für die Herstellung eines Medikaments zur Behandlung von Sehkraftverlust oder Blindheit in einem Säuger.

2. Verwendung von hNT-Neuronen nach Anspruch 1, wobei der Sehkraftverlust durch ein Glaukom verursacht wird.

3. Verwendung von hNT-Neuronen nach Anspruch 1 oder 2, wobei hNT-Neuronen zur Injektion in das Auge sind.

4. Verwendung von hNT-Neuronen nach Anspruch 3, wobei die hNT-Neuronen zusätzlich zur Injektion in den visuellen Cortex sind.

5. Verwendung von hNT-Neuronen nach Anspruch 1, wobei die hNT-Neurouen zur Injektion in den visuellen Cortex sind.

6. Verwendung von hNT-Neuronen nach einem der vorhergehenden Ansprüche, wobei der Säuger zusätzlich mit einem immunsupprimierenden Wirkstoff zu behandeln ist.

7. Verwendung von hNT-Neuronen nach einem der Ansprüche 1 oder 3 bis 6, wobei der Sehverlust verursacht wird durch Sehnervscheide-Meningioma und -Glioma, Graves' Ophthalmopathie, benigne oder maligne Orbitaltumore, metastasische Läsionen, Tumore, die von den benachbarten Sinus paranasales oder der mittleren Schädelgrube entstehen, Riesenhypophysenadenome, Gehimtumore oder -Abszesse, cerebrales Trauma oder Hämorrhagie, Meningitis, arachnoidale Adhäsionen, Pseudotumor cerebri, Sinus cavernosus-Thrombose, Durasinus-Thrombose, Encephalitis, platzbeanspruchende Gehirnläsionen, schwere hypertensive Erkrankung oder pulmonales Emphysem.

8. Verwendung von hNT-Neuronen nach Anspruch 1, wobei der Sehkraftverlust durch retinale Degeneration verursacht wird.

9. Verwendung von hNT-Neuronen nach Anspruch 8, wobei die Degeneration altersbedingte Makuladegeneration ist.

## Revendications

1. Utilisation de cellules souches de neurones hNT dans la fabrication d'un médicament pour le traitement de la perte de la vue ou de la cécité chez un mammifère.

2. Utilisation de neurones hNT selon la revendication 1 dans laquelle la perte de la vue est provoquée par un glaucome.

3. Utilisation de neurones hNT selon la revendication 1 ou 2 dans laquelle les neurones hNT sont destinés à être injectés dans l'oeil.

4. Utilisation de neurones hNT selon la revendication 3 dans laquelle les neurones hNT sont en outre destinés à être injectés dans le cortex visuel.

5. Utilisation de neurones hNT selon la revendication 1 dans laquelle les neurones hNT sont destinés à être injectés dans le cortex visuel.

6. Utilisation de neurones hNT selon l'une quelconque des revendications précédentes dans laquelle le mammifère est également traité avec un médicament immunosuppresseur.

7. Utilisation de neurones hNT selon l'une quelconque des revendications 1 ou 3 à 6 dans laquelle la perte de la vue est provoquée par un méningiome et un gliome de la gaine du nerf optique, une ophtalmopathie de Graves, des tumeurs orbitales bénignes ou malignes, des lésions métastatiques, des tumeurs originaires des sinus paranasaux adjacents ou de la fosse crânienne moyenne, des adénomes pituitaires géants, des tumeurs ou des abcès cérébraux, un traumatisme ou une hémorragie cérébral, une méningite, des adhérences arachnoïdiennes, une pseudotumeur cérébrale, une thrombose du sinus caverneux, une thrombose du sinus dural, une encéphalite, des lésions cérébrales massives, une maladie hypertensive sévère ou un emphysème pulmonaire.

8. Utilisation de neurones hNT selon la revendication 1 dans laquelle la perte de la vue est due à une dégénérescence rétinienne.

9. Utilisation de neurones hNT selon la revendication 8 dans laquelle la dégénérescence est une dégénérescence maculaire liée à l'âge.
